# FASCICULE DE BREVET EUROPEEN

(11) **EP 0 539 451 B1**
(45) Date de publication et mention de la délivrance du brevet: **28.09.1994**
(21) Numéro de dépôt: 91913249.8
(22) Date de dépôt: 15.07.1991
(51) Int. Cl.: C07K 5/06

(54) **VINYLSULFONYL PRISTINAMYCINE ET SA PREPARATION**
VINYLSULFONYL PRISTINAMYCINE UND DEREN HERSTELLUNG
VINYLSULPHONYL PRISTINAMYCIN AND PREPARATION THEREOF

(30) Priorité: 16.07.1990 FR 9009035
(43) Date de publication de la demande: 05.05.1993
(73) Titulaire: RHONE-POULENC RORER S.A., 92160 Antony (FR)
(72) Inventeur: RADISSON, Xavier, F-69006 Lyon (FR)
(74) Mandataire: Savina, Jacques
(86) Numéro de dépôt international: FR9100581
(87) Numéro de publication internationale: WO9201708

(56) Documents cités:
- EP-A- 0 135 410
- EP-A- 0 252 720
- EP-A- 0 298 177

## Description

La présente invention concerne un nouveau dérivé de la pristinamycine II_{B} de formule : 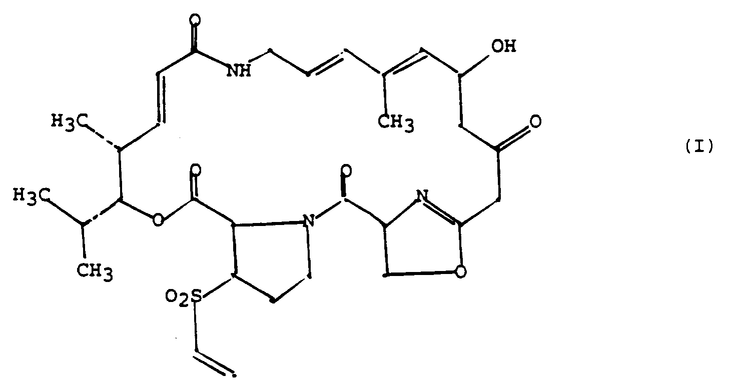
ainsi que leur préparation.

Des dérivés de la pristinamycine II_{B} ont été antérieurement décrits dans les brevets américains US-A-4 590 004 et US-A-4 668 669. Ces produits ont la propriété de synergiser l'activité antimicrobienne de la pristinamycine I_{A}.

Les sulfones vinyliques sont des produits utiles comme intermédiaires de synthèse, particulièrement dans la mise en oeuvre de réactions de Michaël. Leur préparation s'effectue généralement en plusieurs étapes, parmi lesquelles intervient une étape d'oxydation et souvent l'isolement d'une sulfone intermédiaire, puisque la préparation s'effectue dans la plupart des cas en passant par un thiol.

Il a été trouvé que l'on pouvait obtenir la sulfone vinylique de formule (I) en une étape d'oxydation avec un rendement élevé, après avoir introduit le soufre proprement fonctionnalisé en β.

Selon l'invention, la sulfone vinylique dérivée de la pristinamycine II_{B} peut être préparée par oxydation d'une (dialcoylamino-2 éthyl)thio-26 pristinamycine II_{B} de formule générale : 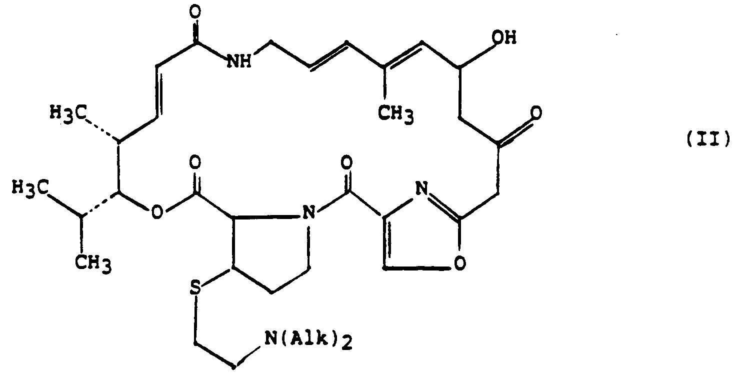
dans laquelle Alk est un radical alcoyle contenant 1 à 4 atomes de carbone en chaîne droite ou ramifiée, par 4 à 6 équivalents d'eau oxygénée, en présence d'un catalyseur tel que le molybdate d'ammonium ou le tungstate de sodium.
Cette nouvelle technique présente l'avantage d'être très douce et très spécifique. Elle put donc être appliquée à des molécules fragiles et comportant de nombreuses fonctions susceptibles d'être altérées par la réaction, comme c'est le cas pour la composante II de la pristinamycine.
La réaction s'effectue dans un alcool code par exemple l'éthanol, le méthanol ou l'isopropanol, à une température comprise entre -25 et +25°C. La quantité de catalyseur exprimée en % molaire, est introduite à raison de 1 à 7% du thioéther de départ.
Il est indispensable d'opérer en présence d'un large excès d'eau oxygénée : à raison de 4 à 6 équivalents par mole de sulfure investie dans la réaction. De préférence on opère en présence de 5 équivalents d'oxydant.
Dans le cas d'une réaction catalysée par le tungstate de sodium, il est entendu que la réaction put être mise en oeuvre à basse température, mais il est nécessaire que la fin de la réaction soit réalisée à une température supérieure ou égale à 0°C.

Un autre intérêt du nouveau procédé selon l'invention est de permettre l'obtention de vinylsulfones dérivées de la pristinamycine II_{B} avec des rendements élevés et sans purification ultérieure.

La (dialcoylamino-2 éthyl)thio-26 pristinamycine II_{B} de formule générale (II) peut être obtenue code décrit dans le brevet US-A-4 590 004.

La sulfone vinylique selon l'invention est utile comme intermédiaire pour la préparation de dérivés de la pristinamycine II_{B} de formule générale : 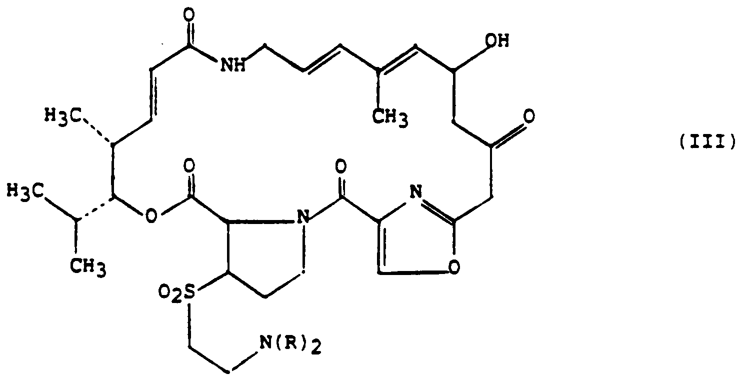
dans laquelle R est un radical alcoyle droit ou ramifié contenant 1 à 10 atomes de carbone, par action de l'amine de formule générale :

H-N(R)₂ (IV)

dans laquelle R est défini comme ci-dessus.

La réaction s'effectue généralement dans un solvant chloré, comme par exemple le chlorure de méthylène, à une température comprise entre 0 et 25°C.

Les dérivés de là pristinamycine de formule générale (III) sont des produits particulièrement interessants qui sont décrits dans le brevet US-A-4 668 669 pour leur activité antibactérienne et leur action synergisante de l'activité antibactérienne de la pristinamycine I_{A} naturelle, de la virginiamycine S et de dérivés solubles de ces produits.

Les exemples suivants donnés à titre non limitatif illustrent la présente invention.

### EXEMPLE 1

65,9 g (0,1 mole) de (diéthylamino-2 éthyl) thio-26 pristinamycine II_{B} sont placés dans 400 cm³ d'éthanol et refroidis à -20°C. L'oxydant (molybdate d'ammonium : 8,65 g, 7 % + eau oxygénée (30 %) : 51,5 cm³, 5 éq.) est additionné en 20 minutes entre -20 et -15°C et l'agitation est poursuivie 40 minutes à -20°C. En 6 minutes, 100 cm³ d'eau sont versés, le bain réfrigérant est enlevé et la solution (0°C) est extraite par 500 cm³ de chlorure de méthylène. La phase organique est lavée par 4 fois 100 cm³ d'eau. Un dosage CLHP de la phase organique donne, à ce stade, un RR = 90 %.
La solution organique est agitée pendant 15 minutes avec 100 cm³ d'une solution 1N d'acide sulfurique, puis lavée par 3 fois 50 cm³ d'eau, séchée sur sulfate de sodium et concentrée à sec. On obtient un solide jaune avec un rendement pondéral de 88,8 % et un titre en vinylsulfonyl-26 pristinamycine II_{B} de 72 % (CLHP) soit un Rendement réel isolé : RR = 63 %.

### EXEMPLE 2

Comme dans l'exemple 1, 3,29 g (5 mmoles) de (diéthylamino-2éthyl) thio-26 pristinamycine II_{B} sont placés dans 20 cm³ d'éthanol et refroidis à -20°C. La solution oxydante (molybdate d'ammonium : 0,346 g, 5,6 % + eau oxygénée (30 %) : 2,05 cm³, 4 éq.) est additionnée en 5 minutes en maintenant la température entre -15°C et -20°C. Le mélange réactionnel est agité pendant 1 heure à cette température puis 15 minutes entre 0 et 5°C pour fournir la vinylsulfonyl-26 pristinamycine II_{B} avec un rendement de 67 %.

### EXEMPLE 3

1 g (1,51 mmole) de (diéthylamino-2 éthyl) thio-26 pristinamycine II_{B} est agité dans 6,1 cm³ d'éthanol et refroidi à -20°C. L'oxydant [tungstate de sodium dihydraté : 5 mg (1 % molaire) + eau oxygénée (30 %) : 0,856 g, 5 équivalents) est additionné en 2 minutes à -20°C. Après 1 heure à -20°C, le mélange réactionnel est ramené à +22°C et après 2 heures, on obtient un rendement réel en vinylsulfonyl-26 pristinamycine II_{B} : RR = 45 % (dosage CLHP).

### EXEMPLE D'UTILISATION

La diéthylamine (0,0517 g, 0,704 mM) diluée par le chlorure de méthylène à un volume total de 0,5 cm³, est additionnée en 20 minutes à environ 20°C à une solution de vinylsulfonyl-26 pristinamycine II_{B} (0,5 g, titre 87 %, 0,704 mM) dans 5 cm³ de chlorure de méthylène. Le mélange est agité pendant 1 heure 15 minutes, puis concentré à sec à l'évaporateur rotatif pendant 25 minutes. On obtient ainsi un solide (masse totale 0,531 g) contenant la (diéthylamino-2 éthyl) sulfonyl-26 pristinamycine II_{B} titre 59,5 % (RR = 65 %) et de la vinylsulfonyl-26 pristinamycine II_{B} de départ 5,1 % (Taux de transformation TT = 94 % ; RT = 69 %).

## Revendications

1. Nouveau dérivé de la pristinamycine II_{B}, de formule :

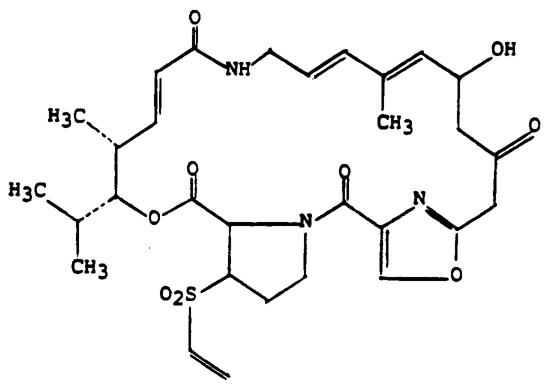

2. Procédé de préparation d'une sulfone vinylique selon la revendication 1, caractérisé en ce que l'on oxyde une (dialcoylamino-2 éthyl)thio-26 pristinamycine II_{B} de formule générale : 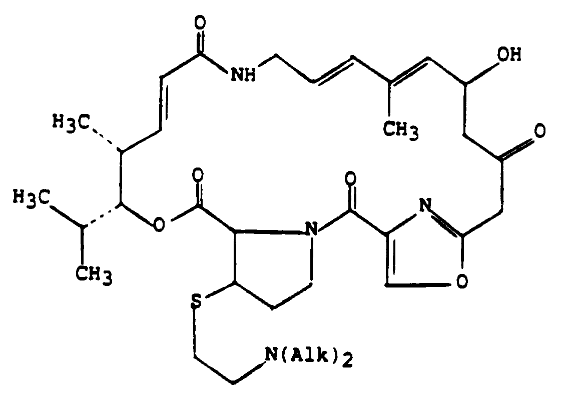 dans laquelle Alk est un radical alcoyle contenant 1 à 4 atomes de carbone en chaîne droite ou ramifiée, par 4 à 6 équivalents d'eau oxygénée, en présence d'un catalyseur tel que le molybdate d'ammonium ou le tungstate de sodium.

3. Procédé selon la revendication 2, caractérisé en ce que le catalyseur est introduit à raison de 1 à 7% molaire par rapport au thioéther de départ.

4. Procédé selon la revendication 2, caractérisé en ce que l'on opère en présence de molybdate d'ammonium.

5. Procédé selon la revendication 2, caractérisé en ce que l'on opère en présence de tungstate de sodium à une température de fin de réaction supérieure à 0°C.

6. Procédé selon la revendication 2, caractérisé en ce que l'on opère dans l'éthanol, le méthanol ou l'isopropanol.

7. Utilisation d'une sulfone vinylique selon la revendication 1 pour la préparation de dérivés de la pristinamycine II_{B} de formule générale : 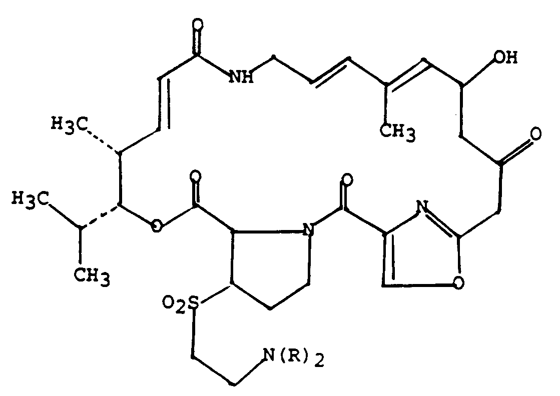 dans laquelle R est un radical alcoyle droit ou ramifié contenant 1 à 10 atomes de carbone, par action d'une amine de formule générale :
H - N(R)₂
dans laquelle R est défini comme ci-dessus.

## Claims

1. New derivative of pristinamycin II_{B}, of formula:

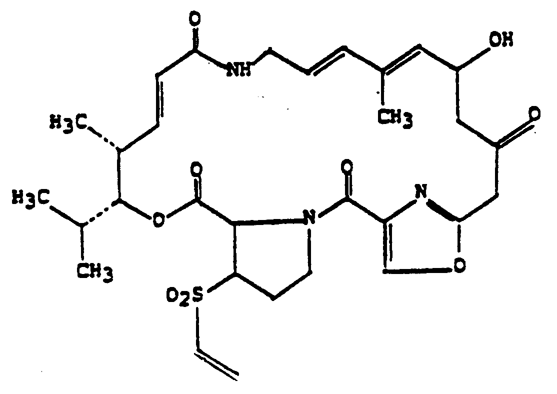

2. Process for the preparation of a vinyl sulphone according to claim 1, characterized in that a 26-(2-dialkylaminoethyl)thio pristinamycin II_{B} of general formula: 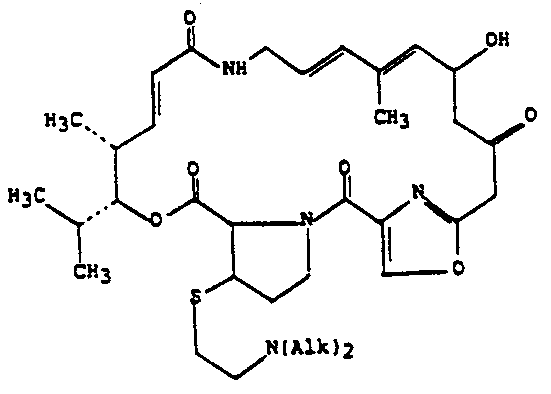 in which Alk is an alkyl radical containing 1 to 4 carbon atoms in a straight or branched chain, is oxidized by 4 to 6 equivalents of hydrogen peroxide in the presence of a catalyst such as ammonium molybdate or sodium tungstate.

3. Process according to claim 2, characterized in that the catalyst is introduced in an amount of 1 to 7 mol% relative to the starting thioether.

4. Process according to claim 2, characterized in that the procedure is carried out in the presence of ammonium molybdate.

5. Process according to claim 2, characterized in that the procedure is carried out in the presence of sodium tungstate at a temperature at the end of the reaction greater than 0°C.

6. Process according to claim 2, characterized in that the procedure is carried out in ethanol, methanol or isopropanol.

7. Use of a vinyl sulphone according to claim 1 for the preparation of derivatives of pristinamycin II_{B} of general formula: 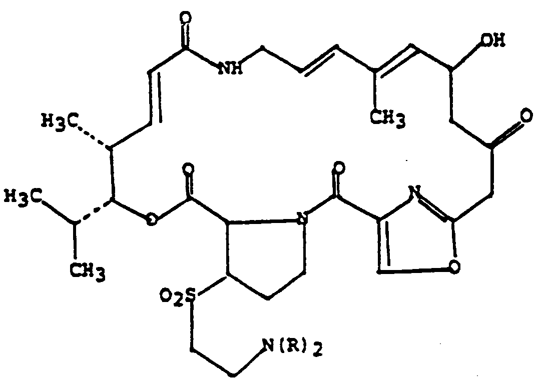 in which R is a straight or branched alkyl radical containing 1 to 10 carbon atoms, by the action of an amine of general formula:
H - N(R)₂
in which R is defined as above.

## Patentansprüche

1. Neue Derivate von Pristinamycin II_{B} der Formel

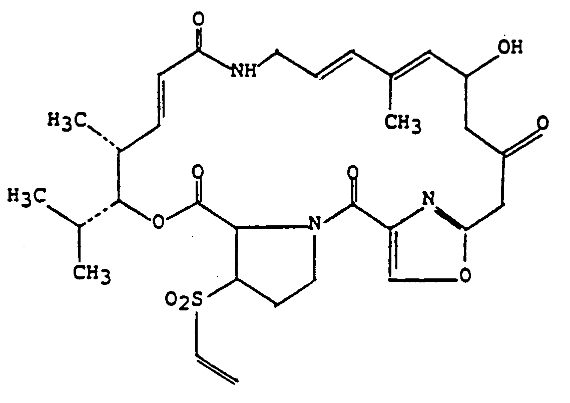

2. Verfahren zur Herstellung eines Vinylsulfones nach Anspruch 1, dadurch gekennzeichnet, daß man ein 26-Thio-(2-dialkylamino-ethyl)-pristinamycin II_{B} der allgemeinen Formel 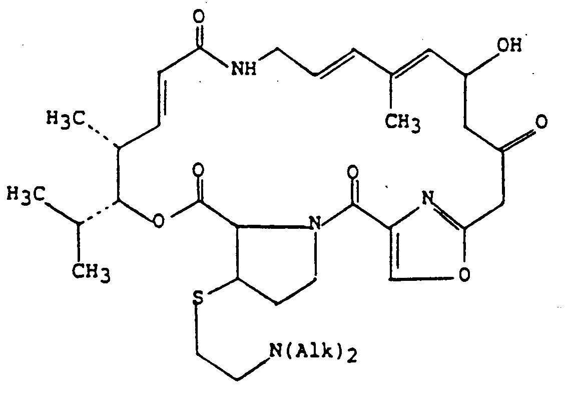 in der Alk einen Alkylrest mit 1 bis 4 Kohlenstoffatomen in gerader oder verzweigter Kette darstellt, mit 4 bis 6 Äquivalenten Wasserstoffperoxid in Anwesenheit eines Katalysators wie Ammoniummolybdat oder Natriumwolframat oxidiert.

3. Verfahren nach Anspruch 2, dadurch gekennzeichnet, daß der Katalysator in einem Verhältnis von 1 bis 7 Molprozent in bezug auf den Thioether als Ausgangsprodukt eingetragen wird.

4. Verfahren nach Anspruch 2, dadurch gekennzeichnet, daß man in Anwesenheit von Ammoniummolybdat arbeitet.

5. Verfahren nach Anspruch 2, dadurch gekennzeichnet, daß man in Anwesenheit von Natriumwolframat bei einer End-Temperatur der Reaktion von höher als 0 °C arbeitet.

6. Verfahren nach Anspruch 2, dadurch gekennzeichnet, daß man in Ethanol, Methanol oder Isopropanol arbeitet.

7. Verwendung eines Vinylsulfones nach Anspruch 1 zur Herstellung des Derivates von Pristinamycin II_{B} der allgemeinen Formel 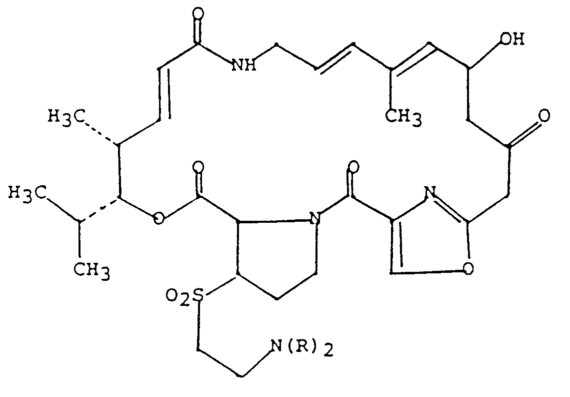 in der R einen geraden oder verzweigten Alkylrest mit 1 bis 10 Kohlenstoffatomen darstellt, durch Einwirkung eines Amines der allgemeinen Formel
H - N(R)₂
in der R wie vorstehend definiert ist.
